# EUROPEAN PATENT SPECIFICATION

(11) **EP 1 244 385 B1**
(45) Date of publication and mention of the grant of the patent: **14.06.2006**
(21) Application number: 00965415.3
(22) Date of filing: 26.09.2000
(51) Int. Cl.: A61B 8/00

(54) **DEVICE FOR APPLYING ENERGY TO A TARGET**
VORRICHTUNG ZUR ANWENDUNG VON ENERGIE AN EINEM ZIEL OBJEKT
DISPOSITIF D'APPLICATION D'ENERGIE SUR UNE CIBLE

(30) Priority: 15.12.1999 US 170776 P
(43) Date of publication of application: 02.10.2002
(73) Proprietor: Super Dimension Ltd., 46733 Herzlia (IL)
(72) Inventor: GILBOA, Pinhas, 34409 Haifa (IL); ZUR, Oded, 42655 Natanya (IL); ESHET, Omer, - (IL)
(74) Representative: Modiano, Micaela Nadia
(86) International application number: PCT/US2000/026322
(87) International publication number: WO 2001/043641

(56) References cited:
- WO-A-96/05768
- US-A- 5 606 975
- US-A- 5 645 065
- US-A- 5 729 129
- US-A- 5 873 822
- US-A- 6 106 517

## Description

### FIELD AND BACKGROUND OF THE INVENTION

The present invention relates to a device for remote application of energy to a target and, more particularly, to a probe, such as a catheter, that includes a sensor for navigating the probe to the target and a force field generator, for depositing the energy at the target, that is configured to interfere minimally with the sensor.

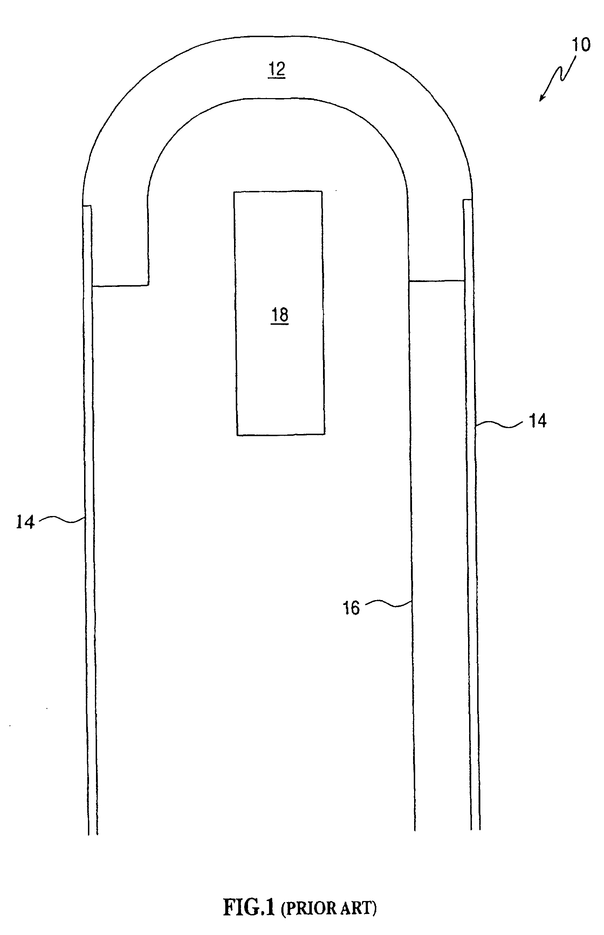

In recent years, several intra-body navigation systems have been developed. Gilboa et al., in WO 00/10456, Ben-Haim et al., in WO 96/05768, and Acker et al., in US 5,558,091 all teach locating systems for tracking the tip of a catheter inside body cavities and blood vessels of a medical or veterinary patient. This is performed by transmitting low frequency electromagnetic waves into the patient's body, incorporating a miniature sensor at the tip of the catheter to measure the field components and calculating the position and orientation of the sensor relative to an external reference frame of coordinates. Martinelli et al., in US 4,821,731, Pfeiler et al., in US 5,'042,486, and Smith et al., in US 5,515,853, teach localization systems based on ultrasonic waves, in which several ultrasound transmitters are placed on the surface of the patient's body and an ultrasound receiver is placed at the tip of the catheter. By measuring the propagation time, the distances between the transmitters and the tip are determined, and the position of the tip is determined by triangulation. Wittkampf, in US 5,697,377, describes a localization system in which three pairs of electrodes are attached to the surface of the patient's body to form three identifiable, mutually orthogonal electrical potentials through the body. An electrode incorporated at the tip of the catheter senses the local electrical potential. From this electrical potential, the position of the catheter electrode is determined.
Intra-body medical and veterinary therapeutic and diagnostic applications have been developed that rely on these navigation systems. For example, Ben-Haim, in US 5,391,199, and Gilboa et al., in WO 00/16684, describe procedures for locating a steerable ablation catheter that is used to treat arrhythmia by ablating a portion of the endocardium using RF current signals. Figure 1 shows an axial cross section of the distal end of one such catheter **10.** The body of catheter **10** is a flexible, substantially cylindrical housing **14.** A metal electrode **12** is mounted at the tip of catheter **10.** An external RF generator is connected to electrode **12** via a conductor 16 that runs along the length of housing **14.** Electrode **12** is secured in contact with the portion of the endocardium to be ablated. That portion of the endocardium then forms the return portion of an electrical circuit. By applying a RF current to conductor **16,** the tissue through which the current is conducted is heated and ablated. An electromagnetic sensor **18,** for determining the position and orientation of electrode **12,** also is incorporated in the tip of catheter **10,** in close proximity to conductor **16.** It is vital that the position and orientation measurements, performed using sensor **18** during the application of the RF signal, be accurate. However, the current conducted via conductor **16** also induces an electromagnetic field. This additional field is sensed by sensor **18,** resulting is a strong jitter of the determined position and orientation. Electronic filtering of the signal from sensor **18** increases the signal-to-noise ratio but delays the response of the navigation system. A similar problem arises when using ultrasonic localization and transmitting ultrasound power while performing ablation or similar procedures such as lithotripsy.

There is thus a widely recognized need for, and it would be highly advantageous to have, a catheter, for the remote effectuation of therapeutic procedures, such as ablation, that apply energy to a target, in which the application of the energy does not interfere with the navigation and localization of the catheter.

### SUMMARY OF THE INVENTION

According to the present invention there is provided a probe, including: (a) a sensor for determining a position of the probe; and (b) a force field generator, disposed relative to the sensor so that there is a node of the force field substantially at the sensor.

According to the present invention there is provided a probe including: (a) a housing; (b) a force field generator depositing energy at a target rigidly attached to the housing; and (c) a sensor for determining the position of the probe and producing a signal, the sensor being rigidly attached to the housing, the signal being subject to perturbation by the force field, the force field generator being disposed symmetrically relative to the sensor, thereby minimizing the perturbation.

According to the present invention there is provided a probe including: (a) a housing having an element of symmetry; (b) a force field generator, for depositing energy at a target rigidly attached to the housing, the force field having a node at least partially within the probe, the force field generator being disposed so that at least a portion of the node is substantially coincident with the element of symmetry; and (c) a sensor determining the position of the probe that is subject to perturbation by the force field, the sensor being rigidly attached to the housing substantially at the node, thereby munimisiag the perturbation.

In the present context, a "probe" is to be understood as any device for remote application of energy to a target Typically, the "probe" of the present invention is a catheter for effecting a medical or veterinary therapeutic procedure; but the scope of the present invention includes any such procedure that must be effected remotely, for example, real-time repair of a nuclear reactor.

The probe includes a sensor for determining the position of the probe, and a force field generator for generating the force field that interacts with the target to deposit the required energy in the target. Because the force field is liable to perturb the signal from the sensor that is representative of the position of the probe, the force field generator is disposed relative to the sensor so as to minimize this perturbation. Preferably, the force field generator is disposed relative to the sensor so that there is a node of the force field substantially at the sensor. A "node" of the force field is a point, line or surface at or along which the amplitude of the force is zero while the generator is operated. The "disposition" of the force field generator that places a node of the force field at the sensor is to be understood to include either a suitable geometric placement of the force field generator relative to the sensor, or a suitable mode of operation of the force field generator, or a suitable combination thereof.

The scope of the present invention includes both oscillatory force fields and static force fields. Examples of oscillatory force fields include electromagnetic and acoustic force fields. Examples of static force fields include DC electrical and magnetic force fields.

Preferably, the sensor is rigidly attached to the probe housing at an element of symmetry of the housing, and the force field generator is disposed symmetrically with respect to the sensor by being rigidly attached to the housing in a manner which is symmetrical with respect to the element of symmetry, so that a node of the force field is substantially coincident with the element of symmetry. For example, the force field generator may include a plurality of elements that are disposed symmetrically with respect to the element of symmetry and with respect to the sensor.

With the force field generator so disposed relative to the sensor, the operation of the force field generator interferes minimally with navigation using the sensor. Using signals from the sensor as taught in the prior art to determine the position of the probe, the probe is placed in proximity to the target. With the probe in proximity to the target, the force field generator is operated to apply energy to the target. Further signals from the sensor are used to determine the position of the probe while the energy is applied to the target, for the purpose of maintaining the probe in proximity to the target while the energy is applied to the target.

### BRIEF DESCRIPTION OF THE DRAWINGS

The invention is herein described, by way of example only, with reference to the accompanying drawings, wherein:
FIG. 1 is an axial cross section of a prior art catheter;
FIG. 2 is an axial cross section of a catheter of the present invention that is used to apply RF energy to a target;
FIG. 3 is a transverse cross section of the catheter of FIG. 2;
FIG. 4 is a transverse cross section of a variant of the catheter of FIGs. 2 and 3;
FIG. 5 is an axial cross section of a catheter of the present invention that is used to apply ultrasound energy to a target;
FIG. 6 is an amplitude contour plot for acoustic signals generated by the catheter of FIG. 5.

### DESCRIPTION OF THE PREFERRED EMBODIMENTS

The present invention is of a probe, such as a catheter, which can be used to apply energy remotely to a target. Specifically, the probe of the present invention is configured so that the application of energy to the target interferes minimally with the navigation of the probe.

The principles and operation of a probe according to the present invention may be better understood with reference to the drawings and the accompanying description.

There are various medical treatments which include localized application of energy to a target. The energy may be either carried to the target via a probe, such as a catheter, or generated at the tip of the probe in close proximity to the target. If the energy is associated with a field, this field may disturb a sensor in the probe that is used to determine the position of the probe relative to the target. The basic concept of the present invention is that if this field is a force field, i.e., a vector field, then the effect of this field on the sensor may be canceled at the probe by intentionally inducing a second field that adds vectorially to the first field at the location of the sensor to cancel the first field there.

One important class of such fields is that of oscillatory fields that carry signals by wave propagation. For example, in some medical treatments, the energy is applied to the target by a coherent wave such as an RF wave, an optical wave produced by a laser, or an ultrasonic acoustic wave. In such a case, the canceling field is induced as a second wave of the same frequency as the first wave, but with a phase shift, relative to the first wave, that cancels the signals, that are carried on the first wave, at the sensor. In general, if a probe includes both a sensor for detennining the position of the probe and a mechanism for inducing a therapeutic signal, and that therapeutic signal interferes with the proper functioning of the sensor, a secondary signal is induced that cancels the primary therapeutic signal at the sensor.

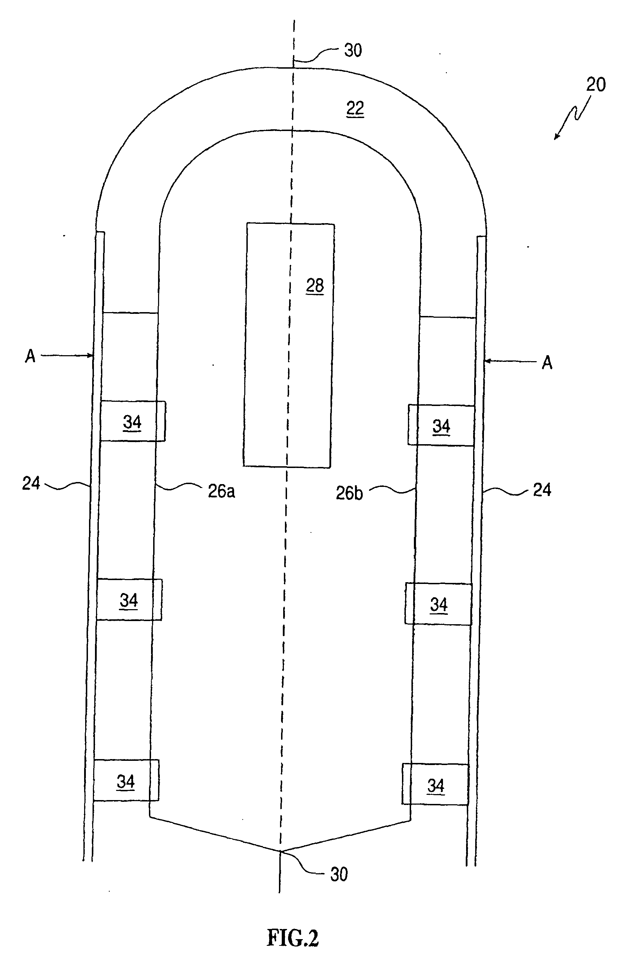

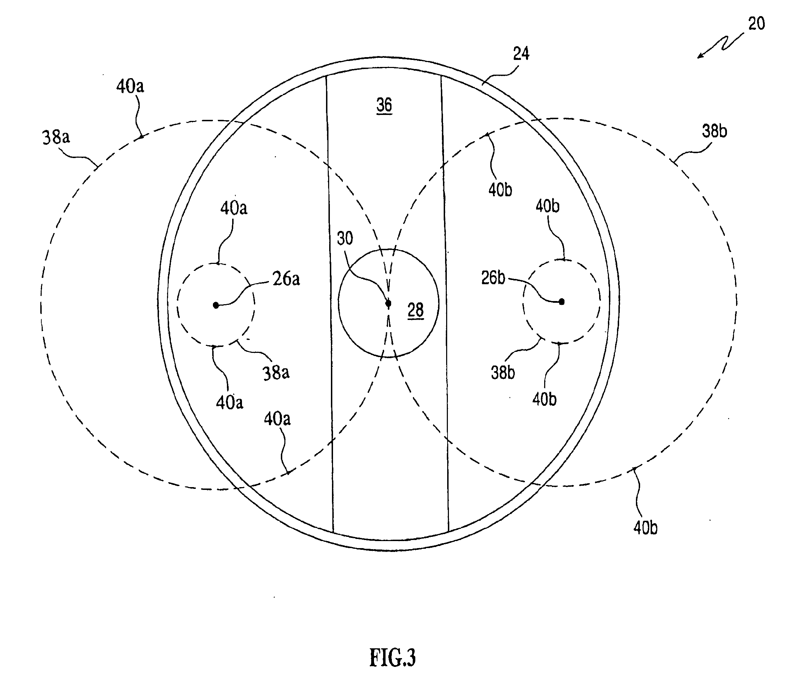

Referring again to the drawings, Figure 2 is an axial cross section of the distal end of a catheter 20 of the present invention, and Figure 3 is a transverse cross section of catheter 20 along cut A-A. As in prior art catheter 10, the body of catheter 20 is a flexible, substantially cylindrical housing 24. A metal electrode 22, similar to electrode 12, is mounted at the tip of catheter 20. An electromagnetic sensor 28, similar in structure and purpose to sensor 18, is incorporated in the tip of catheter 20, aligned along rotational axis 30 of cylindrical housing 24. The difference between catheter 10 and catheter 20 is that catheter 20 has two wires 26a and 26b, on opposite sides of axis 30, for carrying RF current signals to electrode 22. Wires 26a and 26b merge at a junction 32 that is sufficiently far from sensor 28 that RF current flowing in the portion of catheter 20 that is proximal to junction 32 does not affect sensor 28 adversely.

Wires 26a and 26b are parallel to rotational axis 30 of cylindrical housing 24, and equidistant from rotational axis 30. In Figure 3, dashed lines 38a indicate some of the magnetic lines of flux induced by the flow of electrical current in wire 26a, and dashed lines 38b indicate some of the magnetic lines of flux induced by the flow of electrical current in wire 26b. Arrowheads 40 indicate the direction of the associated magnetic force field, when the electrical currents are directed upwards relative to the plane of Figure 3. By symmetry, if the two currents are equal, the two fields cancel each other at axis 30. As a result, the total magnetic field induced in catheter 20 by wires 26a and 26b has a nodal line along axis 30.

Wires 26 are held in place by projections 34 from housing 24. Similarly, sensor 28 is held in place by a projection 36 that spans housing 24. Because sensor 28 is located at a nodal line of the total magnetic field induced by wires 26, the disturbance by this field to the positioning signals produced by sensor 28 is negligible.

The "disposition" of wires 26, that achieves the results of negligible influence on sensor 28, has both a geometric component and an operational component. The geometric component is the siting of wires 26 parallel to, equidistant from and on opposite sides of axis 30. The operational component is the imposition of equal RF currents in wires 26.

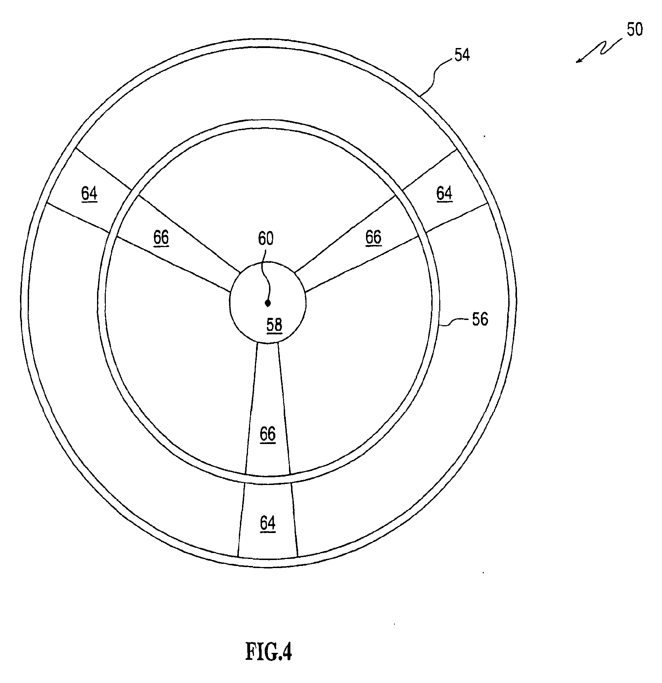

It will be appreciated that the same effect may be achieved using more than two wires 26, as long as those wires are parallel to axis 30, equidistant from axis 30, and spaced at equal azimuthal spacings around axis 30. Figure 4 shows, in transverse cross section, the limiting case of a catheter 50, having a cylindrical housing 54 and a sensor 58 located on rotational axis 60 of housing 54, in which RF current is carried to an electrode (not shown) at the tip of catheter 50 via a conducting nonmagnetic hollow cylinder 56 that is concentric with axis 60. Cylinder 56 and sensor 58 are held in place by projections 64 and 66 from housing 54.

It may not be practical to place wires 26 symmetrically around axis 30. In such a case, the "disposition" of wires 26 includes the selection of (generally unequal) currents to be carried by wires 26 that produce a node of the magnetic field at sensor 28.

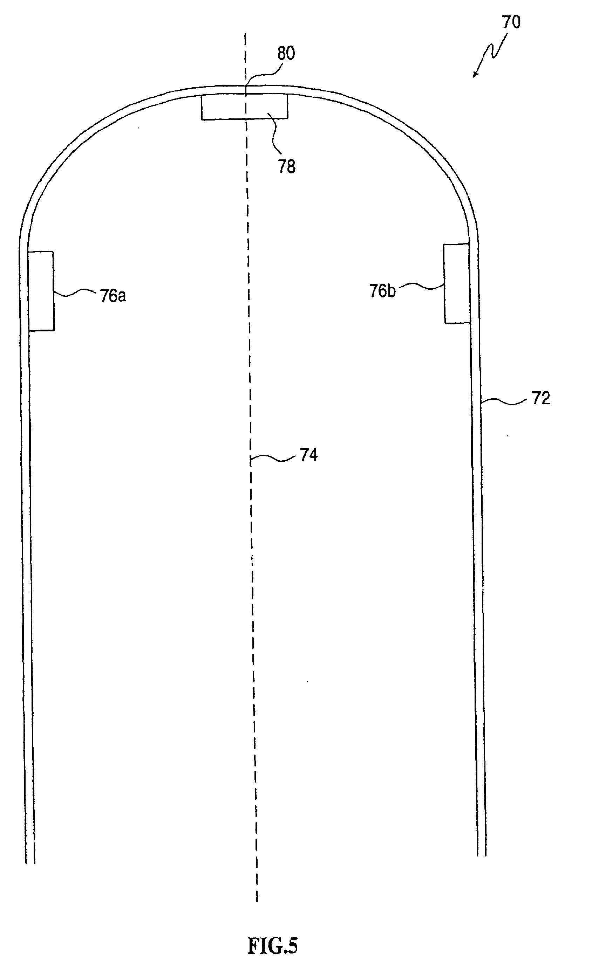

Figure 5 is an axial cross-section of the distal end of a catheter 70 of the present invention for transmitting high-power acoustic waves in a medical or veterinary procedure such as ablation of heart tissue, drilling microchannels in heart muscle tissue, or disintegrating a kidney stone. Catheter **70** is based on a flexible, substantially cylindrical housing **72** that terminates in a tip **80.** Dashed line **74** indicates a plane of reflectional symmetry of housing **72.** Note that plane of symmetry **74** passes through tip **80.** Rigidly attached to housing **72,** on opposite sides of plane of symmetry **74** and equidistant from plane of symmetry **74,** are two ultrasound transmitters **76.** Rigidly attached to housing **72** at tip **80** is an ultrasound receiver **78** that serves as a position sensor for catheter **70,** as described in Martinelli et al., Pfeiler et al. and Smith et al.

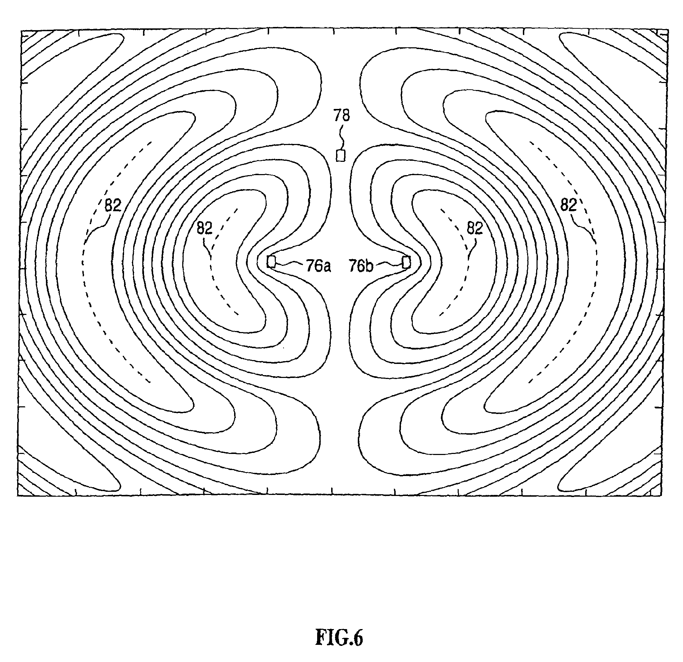

Figure 6 is a conceptual amplitude contour plot, in the plane of Figure 5, for acoustic signals generated by transmitters **76** when transmitter **76** are 180 degrees out of phase. Dashed lines **82** indicate amplitude maxima. Along plane of symmetry **74,** the amplitude vanishes, indicating that plane of symmetry **74** is a nodal plane of the acoustic force field generated by transmitters **76.** Note that in this case, the "disposition" of transmitters **76,** that creates a nodal plane at plane of symmetry **74,** includes both the symmetric placement of transmitters **76** relative to plane of symmetry **74** and the antisymmetric operation of transmitters **76.**

While the invention has been described with respect to a limited number of embodiments, it will be appreciated that many variations, modifications and other applications of the invention may be made.

## Claims

1. A probe (20) comprising:
(a) a housing (24);
(b) a force field (38a, 38b) generator (22, 26a, 26b) depositing energy at a target rigidly attached to said housing (24); and
(c) a sensor (28) for determining the position of the probe and producing a signal, said sensor (28) being rigidly attached to said housing (24), said signal being subject to perturbation by said force field (38a, 38b), said force field (38a, 38b) generator (22, 26a, 26b) being disposed symmetrically relative to said sensor (28), thereby minimizing said perturbation.

2. The probe (20) of claim 1, wherein said force field (38a, 38b) is oscillatory.

3. The probe (20) of claim 2, wherein said force field (38a, 38b) is electromagnetic.

4. The probe (20) of claim 2, wherein said force field (38a, 38b) is acoustic.

5. The probe (20) of claim 1, wherein said force field (38a, 38b) is static.

6. The probe (20) of claim 5, wherein said force field (38a, 38b) is electrical.

7. The probe (20) of claim 5, wherein said force field (38a, 38b) is magnetic.

8. The probe (20) of claim 1, wherein said generator (22, 26a, 26b) includes a plurality of elements (26a, 26b) disposed symmetrically relative to said sensor (28).

9. The probe (20) of claim 1, wherein said signal is representative of a position of the probe (20).

10. A probe (20) comprising:
(a) a housing (24) having an element of symmetry;
(b) a force field (38a, 38b) generator (22, 26a, 26b) for depositing energy at a target, rigidly attached to said housing (24), said force field (38a, 38b) having, during operation, a node at least partially within the probe (20), said force field (38a, 38b) generator (22, 26a, 26b) being disposed so that at least a portion of said node is substantially coincident with said element of symmetry; and
(c) a sensor (28) for determining the position of the probe that is subject to perturbation by said force field (38a, 38b), said sensor (28) being rigidly attached to said housing (24) substantially at said node, thereby minimizing said perturbation.

11. The probe (20) of claim 10, wherein said generator (22, 26a, 26b) includes a plurality of elements (26a, 26b) positioned symmetrically relative to said element of symmetry (30).

## Revendications

1. Sonde (20) comprenant:
(a) un boîtier (24);
(b) un générateur (22, 26a, 26b) de champ de force (38a, 38b) pour déposer de l'énergie sur une cible, rigidement fixé audit boîtier (24) ; et
(c) un capteur (28) pour déterminer la position de la sonde et produire un signal, ledit capteur (28) étant rigidement fixé audit boîtier (24), ledit signal étant sujet à perturbation par ledit champ de force (38a, 38b), ledit générateur (22, 26a, 26b) de champ de force (38a, 38b) étant disposé symétriquement par rapport audit capteur (28), moyennant quoi on minimise ladite perturbation.

2. Sonde (20) selon la revendication 1, dans laquelle ledit champ de force (38a, 38b) est oscillatoire.

3. Sonde (20) selon la revendication 2, dans laquelle ledit champ de force (38a, 38b) est électromagnétique.

4. Sonde (20) selon la revendication 2, dans laquelle ledit champ de force (38a, 38b) est acoustique.

5. Sonde (20) selon la revendication 1, dans laquelle ledit champ de force (38a, 38b) est statique.

6. Sonde (20) selon la revendication 5, dans laquelle ledit champ de force (38a, 38b) est électrique.

7. Sonde (20) selon la revendication 5, dans laquelle ledit champ de force (38a, 38b) est magnétique.

8. Sonde (20) selon la revendication 1, dans laquelle ledit générateur (22, 26a, 26b) comporte une pluralité d'éléments (26a, 26b) disposés symétriquement par rapport audit capteur (28).

9. Sonde (20) selon la revendication 1, dans laquelle ledit signal est représentatif d'une position de la sonde (20).

10. Sonde (20) comprenant :
(a) un boîtier (24) ayant un élément de symétrie ;
(b) un générateur (22, 26a, 26b) de champ de force (38a, 38b) pour déposer de l'énergie sur une cible, ledit champ de force (38a, 38b) ayant, lors du fonctionnement, un noeud au moins partiellement dans la sonde (20), ledit générateur (22, 26a, 26b) de champ de force (38a, 38b) étant disposé de sorte qu'au moins une partie dudit noeud soir sensiblement en coïncidence avec le dit élément de symétrie ; et
(c) un capteur (28) pour déterminer la position de la sonde qui est sujet à perturbation par ledit champ de force (38a, 38b), ledit capteur (28) étant rigidement fixé audit boîtier (24) sensiblement au niveau dudit noeud, moyennant quoi on minimise ladite perturbation.

11. Sonde (20) selon la revendication 10, dans laquelle ledit générateur (22, 26a, 26b) comporte une pluralité d'éléments (26a, 26b) disposés symétriquement par rapport audit élément de symétrie (30).

## Patentansprüche

1. Eine Sonde (20), die folgendes umfasst:
(a) ein Gehäuse (24);
(b) einen Kraftfeld (38a, 38b) Generator (22, 26a, 26b) zur Zuführung von Energie an einem Ziel, der starr mit dem Gehäuse (24) verbunden ist; und
(c) einen Sensor (28) zur Bestimmung der Position der Sonde, wobei der Sensor (28) starr mit dem Gehäuse (24) verbunden ist, wobei das Signal einer Störung durch das Kraftfeld (38a, 38b) unterliegt, wobei der Kraftfeld (38a, 38b) Generator (22, 26a, 26b) in Bezug zu dem Sensor (28) symmetrisch angeordnet ist, wodurch die Störung minimiert wird.

2. Die Sonde (20) nach Anspruch 1, wobei das Kraftfeld (38a, 38b) oszillierend ist.

3. Die Sonde (20) nach Anspruch 2, wobei das Kraftfeld (38a, 38b) elektromagnetisch ist.

4. Die Sonde (20) nach Anspruch 2, wobei das Kraftfeld (38a, 38b) akustisch ist.

5. Die Sonde (20) nach Anspruch 1, wobei das Kraftfeld (38a, 38b) statisch ist.

6. Die Sonde (20) nach Anspruch 5, wobei das Kraftfeld (38a, 38b) elektrisch ist.

7. Die Sonde (20) nach Anspruch 5, wobei das Kraftfeld (38a, 38b) magnetisch ist.

8. Die Sonde (20) nach Anspruch 1, wobei der Generator (22, 26a, 26b) eine Vielzahl von Elementen (26a, 26b) einschließt, die in Bezug zu dem Sensor (28) symmetrisch angeordnet sind.

9. Die Sonde (20) nach Anspruch 1, wobei das Signal die Position der Sonde (20) darstellt.

10. Eine Sonde (20), die folgendes umfasst:
(a) ein Gehäuse (24) mit einem Symmetrieelement;
(b) einen Kraftfeld (38a, 38b) Generator (22, 26a, 26b) zur Zuführung von Energie an einem Ziel, der starr mit dem Gehäuse (24) verbunden ist, wobei das Kraftfeld (38a, 38b) im Betrieb einen Knoten aufweist, der zumindest teilweise innerhalb der Sonde (20) liegt, wobei der Kraftfeld (38a, 38b) Generator (22, 26a, 26b) derart angeordnet ist, dass zumindest ein Teil des Knotens mit dem Symmetrieelement wesentlich übereinstimmt; und
(c) einen Sensor (28) zur Bestimmung der Position der Sonde, der einer Störung durch das Kraftfeld (38a, 38b) unterliegt, wobei der Sensor (28) im Wesentlichen an dem Knoten mit dem Gehäuse (24) starr verbunden ist, wodurch die Störung minimiert wird.

11. Die Sonde (20) nach Anspruch 10, wobei der Generator (22, 26a, 26b) eine Vielzahl von Elementen (26a, 26b) einschließt, die in Bezug zu dem Symmetrieelement (30) symmetrisch angeordnet sind.
